Europäisches Patentamt

European Patent Office

Office européen des brevets

(19)

(11) Veröffentlichungsnummer: **0 106 133**
**B1**

(12) **EUROPÄISCHE PATENTSCHRIFT**

(45) Veröffentlichungstag der Patentschrift: **23.05.90**

(51) Int. Cl.⁵: **A 61 K 7/06**

(21) Anmeldenummer: **83108882.8**

(22) Anmeldetag: **08.09.83**

(54) Konditionierungsmittel für fettiges Haar.

(30) Priorität: **16.09.82 DE 3234365**

(43) Veröffentlichungstag der Anmeldung:
**25.04.84 Patentblatt 84/17**

(45) Bekanntmachung des Hinweises auf die
Patenterteilung:
**23.05.90 Patentblatt 90/21**

(84) Benannte Vertragsstaaten:
**AT BE CH DE FR GB IT LI NL SE**

(56) Entgegenhaltungen:
**DE-A-1 467 825**
**DE-B-1 156 202**
**FR-A- 95 110**
**FR-A-1 563 677**
**FR-A-2 162 464**
**US-A-3 101 301**
**US-A-4 065 422**

(73) Patentinhaber: **Henkel Kommanditgesellschaft
auf Aktien
Postfach 1100 Henkelstrasse 67
D-4000 Düsseldorf-Holthausen (DE)**

(72) Erfinder: **Höffkes, Horst, Dr.
Stettiner Strasse 102
D-4000 Düsseldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Eerfindung sind Haarkonditionierungsmittel in Form einer wäßrigen Dispersion. Solche Zubereitungen gehören zu den sogenannten Haarnachbehandlungsmitteln, da sie nach dem Waschen des Haares angewendet werden und der Pflege dienen.

Nach dem Waschen mit Zubereitungen auf Basis synthetischer oberflächenaktiver Tenside weist das Kopfhaar oft einen kosmetisch unbefriedigenden Zustand auf: Es ist im nassen und im trockenen Zustand schlecht zu kämmen, fühlt sich stumpf an und neigt im trockenen Zustand zur statischen Aufladung, wodurch das Pliegen des frisch gewaschenen Haars verursacht wird.

Man begegnet diesem übelstand dadurch, daß man nach dem Waschen ein Haarkonditionierungspräparat auf das Haar einwirken lößt. Solche Haarkonditionierungspräparate können entweder so aufgebaut sein, daß sie nach einer gewissen Einwirkungszeit wieder aus dem Haar ausgespült werden, wobei ein Teil der Wirkstoffe am Haar adsorbiert bleibt oder sie werden wesentlich verdünnter konzipiert, so daß sie nach der Anwendung im Haar verbleiben. Je nach Zusammensetzung und Anwendungsweise werden solche Haarkonditionierungsmittel als Haarspülung, Haarregenerator, Haarkur, Vitaminkur, Kurpackung, Prisierlotion oder Fönlotion bezeichnet.

Die meisten dieser Präparate sind Emulsionen oder Dispersionen von Fettkomponenten in einen wässrigen Medium, mit einem Gehalt an haarkosmetischen Wirkstoffen wei beispielsweise avivierenden quartären Ammoniumverbindungen, kationischen Polymeren, Proteinderivaten, Vitaminen und anderen bekannten Wirkstoffen. Durch das Waschen mit stark oberflächenaktiven Stoffen wird die Kopfhaut entfettet. Dies führt bei vielen Personen zu einer verstärkten Sebumproduktion und hat ein rasches Nachfetten des Haars zur Folge.

Die Fettkomponenten der Haarkonditionierungsmittel sollen daher der Entfettung der Kopfhaut und des Haares entegegenwirken. Nachteilig ist bei den bekannten Konditionierungsmitteln, daß der Prozeß des Fettig- und Strähnigwerdens des Kopfhaares durch die Vorbelastung mit fettartigen Stoffen noch beschleunigt wird. Das strähnige Aussehen des verfetteten Haars ist darauf zurückzuführen, daß die einzelnen Haarfasern infolge einer gewissen Klebewirkung des aufgenommenen Hautfettes und der daran haftenden natürlich Staub- und Schmutzpartikel sich weitgehend parallel ausrichten und zu Bündeln zusammentreten. Insbesondere bei Personen mit stark nachfettenden Haar wirkt sich die Anwendung der bekannten Konditionierungsmittel in diesem Sinne nachteilig aus.

Es sind schon Versuche gemacht worden, diesem Übelstand dadurch zu begegnen, daß das Haar durch Anwendung von perfluorierten Verbindungen oleophil ausgerüstet wurde. Diese Mittel haben aber den Nachteil, daß sie die Kämmbarkeit des Haares nur wenig verbessern. Es bestand daher die Aufgabe, Haarkonditionierungsmittel zu schaffen, welche die unangenehmen Folgen des Nachfettens der Haare, insbesondere das Fettig- und Strähnigwerden über einen langen Zeitraum vermeiden und darüber hinaus die haarkosmetischen Effekte bekannter Haarkonditionierungsmittel voll zur Geltung bringen.

Diese Aufgabe wurde gelöst durch neue Haarkonditionierungsmittel in Form eine wässrigen Dispersion einer inneren, organischen Phase in einer äußeren wässrigen Phase, dadurch gekennzeichnet, daß die innere, organische Phase zumindest eine Fettkomponente und ein darin gelöstes, fettlösliches Polymerisat, das in Fettalkoholen mit 12 bis 22 Kohlenstoffatomen oberhalb deren Schmelzpunkt zu mindestens 20 Gew.-% löslich ist, enthält. Solche erfindungsgemäßer. Haarkonditionierungsmittel zeigen die überraschende Eigenschaft, daß sie das fettige und strähnige Aussehen des Haares über einen wesentlich längeren Zeitraum verhindern als die bekannten Haarkonditionierungsmittel. Dieser Effekt scheint auf das Zusammenwirken von Fettkomponente und fettlöslichen Polymeren zurückzuführen zu sein. Es wurde gefunden, daß durch den Zusatz des Polymeren zur Fettkomponente deren Klebrigkeit und Tendens zu Aufnahme von Staubpartikeln erheblich gesenkt wird. Dieser überraschende Effekt wurde auch für Mischungen aus Hautfett (Sebum) und fettlöslichen Polymeren experimentell bestätigt.

Die erfindungsgemäßen Haarkonditionierungsmittel enthalten in der inneren, organischen Phase überwiegend die Mischung aus Fettkomponente und fettlöslichem Polymerisat, daneben können aber noch andere Stoffe, zum Beispiel Parfümöle und fettlösliche haarkosmetische Wirkstoffe bekannter Art in der inneren Phase gelöst sein. Bevorzugt besteht die innere organische Phase zu über 90 Gewichtsprozente aus der Mischung aus Fettkomponente und fettlöslichem Polymerisat, wobei die in der inneren Phase gelösten Emulgatoren nicht berücksichtigt sind. Der Anteil des Polymerisats an der Mischung aus Fettkomponente und Polymerisat sollte mindestens 20 Gewichtsprozent betragen. Es hat sich auch als vorteilhaft herausgestellt, wenn die emulgatorfreie innere organische Phase der erfindungsgemäßen Haarkonditionierungsmittel in einem Bereich oberhalb +20°C und unterhalb +35°C schmilzt. Die erfindungsgemäßen Haarkonditionierungsmittel enthalten bevorzugt 0,5 bis 20 Gewichtsprozent der emulgatorfreien, organischen Phase.

Als Fettkomponente können alle für die Anwendung in Haarkonditionierungsmitteln bekannten Fett- und Ölkomponenten eingesetzt werden, zum Beispiel Vaseline, Paraffine, Fettsäuretriglyceride, Fettsäurepartialglyceride, Fettsäurealkylester, Alkylenglycolfettsäureester sowie lineare und verzweigtkettige, natürliche und synthetische Fettalkohole. Bevorzugt geeignete Fettkomponenten weisen eine Schmelzbereich öberhalb +20°C auf, ganz besonders bevorzugt geeignet sind lineare Fettalkohole mit 12 bis 32 Kohlenstoffatomen, insbesondere Cetylalkohol und Stearylalkohol sowie Gemische diese Fettalkohole.

2

Als fettlösliches Polymerisat können alle bekannten Polymerisations- und Polykondensationsprodukte verwendet werden, die aufgrund ihrer Struktur in Fettkomponenten der vorgenannten Art eine hohe Löslichkeit aufweisen. Als Auswahlkriterium ist die Löslichkeit in Fettalkoholen mit 12—22 Kohlenstoffatomen, zum Beispiel in Cetylalkohol oder Stearylalkohol geeignet. In diesen Alkoholen sind die erfindungsgemäß geeigneten Polymerisate oberhalb des Schmelzpunktes dieser Fettalkohole zu mindestens 20 Gewichtsprozente klar und homogen löslich.

Besonders geeignet als Polymere sind zum Beispiel Copolymerisate aus N-Vinylpyrrolidon und Monomeren, die eine lineare Fettalkylkette mit 8 bis 22 Kohlenstoffatomen aufweisen. Als Beispiele für solche Comonomere seien Allyl- und Methallylfettsäureester, Fettalkylvinylether und α-Olefine gennant. Die Fettlöslichkeit solcher Copolymerisate läßt sich durch das Mengenverhältnis der Comonomeren zum N-Vinylpyrrolidon beliebig einstellen. Fettlösliche Copolymere dieser Art sind handelsübliche Produkte. Als Beispiel seien Copolymere aus N-Vinylpyrrolidon und α-Olefinen genannt, die unter den Warenzeichen Ganex®-V-Polymere und Antaron® V der Firma GAF-Corporation im Handel sind.

Die erfindungsgemäßen Haarkonditionierungsmittel werden unter Verwendung bekannter Emulgatoren hergestellt. Geeignete Emulgatoren zur Herstellung der wässrigen Dispersion der organischen Phase sind zum Beispiel nichtionogene Anlagerungsprodukte von Ethylenoxid an Fettalkohole, Fettsäuren, Alkylphenole, Fettsäurepartialglyceride, Sorbitanfettsäureester, Fettsäureamide, Fettsäurealkanolamide, Fettamine, natürliches oder hydriertes Rizinusöl. Als Emulgatorn eignen sich auch ampholytische Tenside und kationische Tenside. Die kationische Tenside sind bevorzugt quartäre Ammoniumverbindungen mit einem linearen Fettalkylrest mit 8—22 Kohlenstoffatomen. Solche Produkte werden den bekannten Haarkonditionierungsmitteln auch als avivierende, antistatische Komponente zugesetzt.

Weiterhin können die erfindungsgemäßen Haarkonditionierungsmittel übliche Hilfsmittel wie Konservierungsstoffe, Farb- und Duftstoffe sowie übliche haarkosmetische Zusätze enthalten. Solche üblichen Zusätze zur Erzielung bestimmter kosmetischer Effekte sind zum Beispiel die bereits erwähnten, auch als Emulgatoren verwendbaren quartären Ammoniumverbindungen mit wenigstens einem linearen Fettalkylrest mit 8—22 Kohlenstoffatomen. Weitere, eine Haaravivage und eine Verbesserung der Kämmbarkeit bewirkenden Additive sind wasserlösliche kationische Polymere, zum Beispiel Polysaccharide wie Cellulose-, Stärke- oder Galaktomannan-Derivate mit kationischen, quartären Ammoniumgruppen. Auch wasserlösliche Proteine oder Proteinderivate, Pantothensäure, Vitamine, sebostatische, antistatische, antimikrobielle und desodorierende Verbindungen können den Haarkonditionierungsmitteln zugesetzt werden.

Die erfindungsgemäßen Haarkonditionierungsmittel zeigen besonders bei Personen, die zu starkem Fetten des Haupthaares neigen, eine hervorragende Wirkung, da in diesem Falle die Verhinderung des fettigen und strähnigen Aussehens der Haare besonders erwünscht ist. Darüber hinaus können den erfindungsgemäßen Haarkonditionierungsmittel auch besonders stark rückfettende oder avivierende Komponenten enthalten, ohne daß die Gefahr einer zu schnellen Verschlechterung des haarkosmetischen Zustandes des damit behandelten Haares besteht, da die Klebrigkeit und die damit verbundene Aufnahme von Schmutz- und Staubpartikeln über einen längeren Zeitraum hintangehalten wird.

Die folgenden Beispiele sollen den Gegenstand der Erfindung näher erläutern ohne ihn hierauf zu beschränken.

## Beispiele

Da sich der haarkosmetische Effekt der erfindungsgemäßen Haarkonditionierungsmittel am Haar nicht zahlenmäßig erfassen läßt, wurde die klebrigkeitsvermindernde Wirkung von fettlöslichen Polymeren in einem Modellversuch demonstriert. Hierzu wurde die Staubanfälligkeit von synthetischem Sebum und von Mischungen dieses Fettes mit der Fettphase eines erfindungsgemäßen Konditionierungsmittels und eines von fettlöslichen Polymeren freien Konditionierungsmittels nach folgender Methode bestimmt:

## Versuchanordnung

Aluminiumschälchen mit einer Bodenfläche von ca. 20 cm$^2$ wurden am Boden mit einer dünnen Schicht der Fettmischungen A, B, C und D (Tabelle I) belegt und ausgewogen Dann wurden auf die Fettoberfläche jeweils 100 mg einer hydrophobierten pyrogenen Kieselsäure (Aerosil® R 972, Firma Degussa) gleichmäßig aufgestreut. Nach einer Lagerung von 30 Minuten bei 20°C wurden die Aluminiumschälchen umgestürzt und durch leichtes Klopfen und Blasen mit einem trockenen Luftstrom der nicht anhaftende Kieselsäurestab entfernt. Danach wurden die Schälchen erneut ausgewogen. Die durch Gewichtsdifferenz ermittelte Menge anhaftender Kieselsäure ist ein Maß für die Klebrigkeit der Fettphase.

## Ergebnis

Das aus Tabelle I ersichtliche Ergebnis zeigt, daß durch Zugabe des fettlöslichen Polymeren Ganex®-Polymer V—220 (GAF-Corporation) die Klebrigkeit der Fettphasen stark erniedrigt wird.

3

# EP 0 106 133 B1

TABELLE I

| | | Fettphase | | anhaftende Kieselsäure |
|---|---|---|---|---|
| A | | Synthetisches Sebum* | 100 Gew.% | 10 mg |
| B | | Synthetisches Sebum* | 50 Gew.% | |
| | | Fettalkohol $C_{16-18}$ | 40 Gew.% | |
| | | Fettalkohol $C_{16-18}$ | | 4,5 mg |
| | | + 12 Mol Ethylenoxid | 10 Gew.% | |
| C | | Synthetisches Sebum* | 50 Gew.% | |
| | | Fettalkohol $C_{16-18}$ | 20 Gew.% | |
| | | Fettalkohol $C_{16-18}$ | | 2,0 mg |
| | | + 12 Mol Ethylenoxid | 5 Gew.% | |
| | | Ganex® Polymer V—220 | 25 Gew.% | |
| D | | Fettalkohol $C_{16-18}$ | 40 Gew.% | |
| | | Fettalkohol $C_{16-18}$ | | 1,1 mg |
| | | + 12 Mol Ethylenoxid | 10 Gew.% | |
| | | Ganex® Polymer V—220 | 50 Gew.% | |

\* Das synthetische Sebur wurde nach F. U. Wells: Soap, Pefumery and Cosmetics, Juli 1961, S. 638, Rezeptur für Synthetic Sebum hergestellt.

Im folgenden werden Rezepturbeispiele für erfindungsgemäße Haarkonditionierungsmittel gegeben.

1.  Haarspülung

| | | |
|---|---|---|
| Myristylalkohol | 0,35 | Gewichtsprozent |
| Cetylalkohol | 0,65 | " |
| Stearylalkohol | 0,65 | " |
| Ganex®-Polymer V—220 | 2,0 | " |
| Cetyltrimethylammoniumchlorid | 0,5 | " |
| Parfümöl | 0,2 | " |
| Konservierungsmittel | 0,3 | " |
| Wasser | 95,35 | " |

2.  Haakur, ausspülbar

| | | |
|---|---|---|
| Cetylalkohol | 2,1 | Gewichtsprozent |
| Stearylalkohol | 2,1 | " |
| Ganex®-Polymer V—220 | 5,0 | " |
| Fettalkohol $C_{16-18}$ + 12 Mole Ethylenoxid | 1,3 | " |
| Cetyltrimethylammoniumchlorid | 0,5 | " |
| Parfümöl | 0,2 | " |
| Konservierungsmittel | 0,3 | " |
| Wasser | 88,5 | " |

3. Konditionsierungsmittel, im Waar verbleibend (Fönlotion)

| | | |
|---|---|---|
| Myristiylalkohol | 0,5 | Gewichtsprozent |
| Fettalkohol C$_{16-18}$ +12 Mole Ethylenoxid | 0,1 | „ |
| Ganex®-Polymer V—220 | 5,0 | „ |
| Cetyltrimethylammoniumchlorid | 0,5 | „ |
| Parfümöl | 0,2 | „ |
| Konservierungsmittel | 0,3 | „ |
| Wasser | 97,9 | „ |

## Patentansprüche

1. Haarkonditionierungsmittel in Form einer wäßrigen Dispersion, dadurch gekennzeichnet, daß die innere, organische Phase eine Mischung aus einer Fettkomponente und einem darin löslichen Polymerisat, das in Fettalkoholen mit 12 bis 22 Kohlenstoffatomen oberhalb deren Schmelzpunkt zu mindestens 20 Gewichtsprozent löslich ist, enthält.

2. Haarkonditionierungsmittel nach Anspruch 1, dadurch gekennzeichnet, daß die emulgatorfreie innere Phase mehr als 50 Gewichtsprozent, bevorzugt mehr als 90 Gewichtsprozent, der Mischung aus Fettkomponente und Polymerisat enthält, und daß diese Mischung mindestens 20 Gewichtsprozent des Polymerisats enthält.

3. Haarkonditionierungsmittel, nach Anspruch 1 und 2, dadurch gekennzeichnet, daß die emulgatorfreie, innere Phase einen Schmelzbereich zwischen 20°C und 35°C aufweist.

4. Haarkonditionierungsmittel nach Anspruch 1 bis 3, dadurch gekennzeichnet, daß die Fettkomponente einen Fettalkohol mit 12 bis 22 Kohlenstoffatomen darstellt.

5. Haarkonditionierungsmittel nach Anspruch 1 bis 4, dadurch gekennzeichnet, daß das fettlösliche Polymerisat eine lineares Copolymerisat aus N-Vinylpyrrolidon und einem Monomeren mit einer linearen Fettalkylkette mit 8 bis 22 Kohlenstoffatomen, ausgewählt unter Allyl- und/oder Methallylfettsäureestern, Fettalkylvinylethern und/oder α-Olefinen ist.

## Revendications

1. Compositions pour le conditionnement des cheveux sous forme d'une dispersion aqueuse, caractérisées en ce que la phase interne organique est constituée d'un mélange d'un composant gras et d'un polymère soluble dans celui-ci, qui est soluble dans les alcools gras comportant 12 à 22 atomes de carbone, à raison d'au moins 20% en poids au delà du point de fusion desdits alcools gras.

2. Compositions pour le conditionnement des cheveux selon la revendication 1, caractérisées en ce que la phase interne exempte d'émulsifiants renferme plus de 50 pour cent en poids, de préférence plus de 90 pour cent en poids, du mélange du composant gras et du polymère, et en ce que mélange renferme au moins 20 pour cent en poids du polymère.

3. Compositions pour le conditionnement des cheveux selon la revendications 1 et 2, caractérisées en ce que la phase interne exemte d'émulsifiants présente un intervalle de fusion compris entre 20 et 35°C.

4. Compositions pour le conditionnement des cheveux selon la revendications 1 et 3, caractérisées en ce que le composant gras est un alcool gras comportant 12 à 22 atomes de carbone.

5. Compositions pour le conditionnement des cheveux selon les revendications 1 et 4, caractérisées en ce que le polymère liposoluble est un copolymère linéaire constitué de N-vinylpyrrolidone et d'un monomère avec une chaîne linéaire d'alkyle gras comportant 8 à 22 atomes de carbone, polymère choisi parmi les esters d'acides gras allyliques et/ou méthallyliques, les éthers vinyliques d'alkyle gras et/ou les α-oléfines.

## Claims

1. A hair conditioner in the form of an aqueous dispersion, characterized in that the inner organic phase contains a mixture of a fatty component and a polymer soluble therein which forms at least 20% by weight solutions in C$_{12-22}$ fatty alcohols above their melting point.

2. A hair conditioner as claimed in claim 1, characterized in that the emulsifier-free inner phase contains more than 50% by weight and preferably more than 90% by weight of the mixture of fatty component and polymer and in that this mixture contains at least 20% by weight of the polymer.

3. A hair conditioner as claimed in claims 1 and 2, characterized in that the emulsifier-free inner phase has a melting range of 20 to 35°C.

4. A hair conditioner as claimed in claims 1 to 3, characterized in that the fatty component is a $C_{12-22}$ fatty alcohol.

5. A hair conditioner as claimed in claims 1 to 4, characterized in that the fat-soluble polymer is a linear copolymer of N-vinyl pyrrolidone and a monomer with a linear $C_{8-22}$ fatty alkyl chain selected from allyl and/or methallyl fatty acid esters, fatty alkyl vinyl ethers and/or α-olefins.